# EUROPÄISCHE PATENTSCHRIFT

(11) **EP 1 292 246 B1**
(45) Veröffentlichungstag und Bekanntmachung des Hinweises auf die Patenterteilung: **11.10.2006**
(21) Anmeldenummer: 01947293.5
(22) Anmeldetag: 18.05.2001
(51) Int. Cl.: A61F 2/06

(54) **RADIAL EXPANDIERBARE GEFÄSSSTÜTZE**
RADIALLY EXPANDABLE VASCULAR STENT
TUTEUR VASCULAIRE RADIALEMENT EXTENSIBLE

(30) Priorität: 22.05.2000 WO PCT/EP00/04658
(43) Veröffentlichungstag der Anmeldung: 19.03.2003
(73) Patentinhaber: Eurocor GmbH, 53113 Bonn (DE)
(72) Erfinder: ORLOWSKI, Michael, 53173 Bonn (DE)
(74) Vertreter: Thiel, Christian
(86) Internationale Anmeldenummer: PCT/EP2001/005736
(87) Internationale Veröffentlichungsnummer: WO 2001/089414

(56) Entgegenhaltungen:
- WO-A-99/17680
- FR-A- 2 758 253
- US-A- 5 913 895

## Beschreibung

Die Erfindung betrifft eine radial aufweitbare Gefäßstütze zur Verwendung zum Offenhalten von Blutgefäßen oder sonstigen Organwegen in menschlichen oder tierischen Körpern. Diese gitterförmige Gefäßstütze besteht aus mehreren rohrförmigen Elementen mit einer im wesentlichen zickzackförmigen Ringstruktur von geringer Breite, deren Ringe mit hantel- oder S-förmigen Stegen miteinander verbunden sind.

In der Patentschrift EP 335 341 B1 sind Gefäßstützen beschrieben, die aus langgestreckten Gliederpaaren gebildet sind. Diese Gefäßstützen werden beispielsweise in verengte oder andere Körpergefäße implantiert, um diese nach Ballondilatation dauerhaft offen zu halten. Dabei werden die Gefäßstützen in ihrem Durchmesser aufgeweitet und verkürzen sich in ihrer seitlichen Länge. Diese Verkürzung ist in der Regel unerwünscht, da dies eine Fehlpositionierung der Gefäßstütze verursachen kann. Die bekannten Gefäßstützen passen sich Bögen oder Kurven im Gefäßverlauf relativ schlecht oder gar nicht an, so daß zusätzliche Biegeelemente vorgesehen werden müssen.

Die bekannten Gefäßstützen weisen starre röhrenförmige Abschnitte auf, die durch gelenkige Verbindungsstücke etwas biegsamer miteinander verbunden sind. Dabei können aber in diesen Bereichen, wegen der besonderen Wandbeanspruchung bei jeder Gefäßbewegung, Hypertrophien der Gefäßwand auftreten. Andere bekannte Gefäßstützen weisen besonders bei Aufdehnung im Bereich ihres Maximaldurchmessers eine erheblich Verkürzung auf.

Gefäßstützen mit gleichsinnig gerichteten bogenförmigen Stegen zwischen den zickzackförmigen Ringelementen sind beispielsweise aus der Patentschrift DE 197 40 506 A1 bekannt. Durch die zahlreichen Stege zwischen den Ringelementen ist jene Gefäßstütze jedoch sehr steif und unflexibel, was zu einem Fehlschlag bei Implantationsversuchen bei kurvigem Gefäßverlauf führen kann.

Durch die zahlreichen bogenförmigen Stege können auch unbeabsichtigt Seitenäste des Gefäßsystems verschlossen werden, die offen bleiben sollen.

Durch die gleichsinnig gerichteten, bogenförmigen Stege auf der Zirkumferenz besteht auch der Nachteil, daß diese Gefäßstütze bei kurvigem Gefäßverlauf leicht knickt und das Gefäß teilweise oder ganz verschließt, welches mit der Gefäßstütze offengehalten werden sollte.

US-A- 5 913 895 offenbart eine Gefäßstüke mit randständigen Ringelementen, die durch hantelförmige Biegeelemente Verbunden sind und Zentralständige Ringelemente, die durch S-förmige Biegeelemente verbunden sind.

Aufgabe der vorliegenden Erfindung ist es, eine radial aufweitbare Gefäßstütze zu schaffen, die während ihrer Aufweitung keine oder nur eine geringe Verkürzung erfährt, bei besserer Kurvengängigkeit weniger leicht knickt und zugleich eine ausreichende Radialfestigkeit aufweist. Auch soll die Gefäßstütze im aufgeweiteten Zustand zwischen den einzelnen Streben genügend große Querschnitte für das Offenbleiben von Seitenastabgängen des Gefäßsystems aufweisen.

Diese Aufgabe für die erwähnte Gefäßstütze wird dadurch gelöst, daß die Gefäßstütze eine Vielzahl von miteinander durch Biegeelemente flexibel verbundene Ringelemente aufweist, welche eine Gefäßstütze mit einem proximalen und einem distalen Ende und einer Längsachse definieren, wobei die Ringelemente quer zur Längsachse der Gefäßstütze nebeneinander angeordnet sind, und jeweils durch Biegeelemente miteinander verbunden sind, und wobei am proximalen und am distalen Ende der Gefäßstütze, jeweils wenigstens zwei randständige Ringelemente untereinander durch ein Paar hantelförmige Biegeelemente verbunden sind und zentralständige Ringelemente untereinander und mit randständigen Ringelementen durch jeweils zwei S-förmige Biegeelemente verbunden sind, wobei zumindest die zentralständigen Ringelemente zickzackförmig ausgebildet sind.

Bei radialer Aufweitung der Gefäßstütze strecken sich die Biegeelemente entsprechend der seitlichen Verkürzung der zickzackförmigen Ringelemente in der Längsachse und vermeiden oder verringern so eine Gesamtverkürzung der Gefäßstütze. Die S-förmigen Stege sind erfindungsgemäß bei den zentralen Ringelementen jeweils paarweise angeordnet, so daß sich im zentralen Bereich eine besonders hohe Flexibilität ergibt. Die S-förmigen Stege sind zweckmäßigerweise paarweise so angeordnet, daß sie sich auf der Zirkumferenz der Gefäßstütze gegenüberstehen. Dabei können die S-förmigen Biegeelemente gleichsinnig oder gegensinnig-angeordnet sein; die gleichsinnige Anordnung ist wegen ihrer höheren Stabilität bevorzugt, jedoch können zur Erhöhung der Flexibilität auch gegensinnige Anordnungen herangezogen werden.

Am proximalen und an distalen Ende der erfindungsgemäße Gefäßstütze befinden-sich-jeweils zwei oder mehr randständige Ringelemente, die untereinander durch ein Paar hantelförmige Biegeelemente verbunden sind. Zweckmäßigerweise sind diese randständigen Ringelemente schlangelinienförmig ausgebildet, wobei zwei benachbarte randständige Ringelemente zweckmäßigerweise mit ihren Bögen aufeinanderweisen. Im Bereich dieser aufeinanderweisenden Bögen befinden sich auf einander gegenüberliegenden Seiten der Zirkumferenz gerade Verbindungsstege von etwa hantelförmigen Design. Die schlagenlinienförmige Ausbildung der randständigen Ringelemente in Verbindung mit den geraden Verbindungs- bzw. Biegeelementen verleiht der Gefäßstütze in ihren Randbereichen eine erhöhte Rigidität, die der Stabilisierung an der Gefäßwand dient und eine zuverlässige Verankerung erlaubt.

Im Sinne der Erfindung bedeutet der Begriff "hantelförmig" in Bezug auf ein Biegeelement eine im wesentlichen gerade ausgerichtete Stegverbindung zwischen den Bögen zweier Ringelemente. Der Begriff "S-förmig" bezeichnet Biegeelemente, die die Form eines aufrechten, liegenden oder schräggestellten S, oder eines spiegelverkehrten S haben. "Gleichsinnig" bedeutet, daß die S-förmigen oder anderen Elemente auf der Oberfläche der Gefäßstütze in der flächigen Darstellung gleichförmig ausgerichtet sind.

Zweckmäßigerweise sind die zickzackförmigen Ringelemente an den Enden jeweils zu Bögen abgerundet. Dabei kann die Breite der zickzackförmigen Ringelemente im Bereich der Bögen größer sein, als im Bereich der Stege.

Ferner kann zur Erhöhung der Stabilität in einzelnen Bereichen insbesondere zu den seitlichen Ende der Gefäßstütze hin, die Breite der Stege und/oder der zickzackförmigen Ringelemente größer sein, als in anderen Bereichen, insbesondere im zentralen Bereich. Andererseits kann aber auch zur Erhöhung der Radialkraft im zentralen Bereich der Gefäßstütze die Breite der Stege und/oder der Bögen der zickzackförmigen Elemente im zentralen Bereich der Gefäßstütze größer sein als an den Enden. Die Ausführungsformen hängen jeweils vom Einsatzzweck, vom Verlauf des Gefäßes, etwaigen Verzweigungen des Gefäßes und dergleichen Faktoren ab. Auf diese Weise läßt sich eine weite Variabilität in Bezug auf Anpassungsfähigkeit und radiale Stabilität erzielen.

Die erfindungsgemäße aufweitbare Gefäßstütze ist zweckmäßigerweise in ihrem zentralen Bereich so aufgebaut, daß die zickzackförmigen Ringelemente mit ihren Bögen gleichsinnig ausgerichtet sind, d. h. alle Bögen auf gleicher Höhe in die gleiche Richtung weisen. Entsprechendes gilt für die Bögen aneinandergrenzender schlangenlinienförmiger und zickzackförmiger Ringelemente. Dies ermöglicht, die S-förmigen Biegeelemente diagonal über die Gefäßstütze versetzt anzuordnen, so daß sich eine Spiralform ausbildet. Hierdurch hat die Gefäßstütze eine von Ringelement zu Ringelement bevorzugte Biegerichtung.

Die erfindungsgemäß eingesetzten S-förmigen Biegeelemente an den zickzackförmigen Ringelementen sind im wesentlichen in vertikaler Richtung angeordnet, d. h. sie verlaufen im wesentlichen senkrecht zu den Stegen der zickzackförmigen Ringelemente. Die dergestalt ausgebildeten Gefäßstützen haben hierdurch eine größtmögliche Flexibilität. Um zusätzliche Stabilität in Längsrichtung zu gewinnen, kann es allerdings sinnvoll sein, die S-förmigen Biegeelemente im wesentlichen parallel zu den Stegen und Bögen der zickzackförmigen Ringelemente auszurichten. Hierdurch wird eine gute Kompensation der Längenverkürzung bei Aufweitung der Gefäßstütze erreicht.

Die Biegeelemente zwischen den einzelnen Ringelementen weisen vorzugsweise einen etwas geringeren Querschnitt auf, als der gerade Stegbereich der zickzackförmigen Ringelemente, zweckmäßigerweise um 10-50% und insbesondere um etwa 30%.

Des weiteren können die zickzackförmigen Ringelemente am Rand und im Mittelbereich der Gefäßstütze unterschiedliche Querschnitte aufweisen. Zur Verbesserung der Stützeigenschaften und Radialfestigkeit im Randbereich weist die Gefäßstütze an beiden Enden eine größere Stegbreite auf. Zur Verbesserung der lokalen Stützeigenschaften im Bereich einer fokalen Gefäßverengung und der Radialfestigkeit weist die Gefäßstütze andererseits nur im Mittelbereich eine größere Stegbreite und/oder eine größeren Querschnitt auf. Der größere Querschnitt im Mittelbereich kann z. B. durch eine weniger Material abtragende Elektropolitur erreicht werden.

Das Biegeverhalten der Gefäßstütze beim Crimpen und bei der Expansion kann durch eine besondere Ausbildung der Bögen der zickzackförmigen Ringelemente, beispielsweise C-förmig, haarnadelförmig oder klammerförmig, weiter verbessert werden, besonders wenn die Breite des C-förmigen oder des klammerförmigen Bogens geringer ist als die des Steges des zickzackförmigen Ringelements.

Als Material für die Gefäßstütze können insbesondere eines oder mehrere biokompatible Materialien aus der Gruppe Niob, Platin, Stahl, Titan, einer Legierung aus Nickel-Titan, Platin-Iridium oder einer Legierung mit mindestens einem dieser Metalle, wie Platin-Iridium mit jeweils geeigneten Gewichtsprozenten, verwendet werden. Soll die Gefäßstütze selbstexpandierbar sein, wird vorzugsweise eine durch Wärmebehandlung temperaturoptimierte Nickel-Titanlegierung (Nitinol) verwendet.

Das Metall kann zur Verbesserung des Einwachsens in die Gefäßwand mit einem biokompatiblen Material oder mit geeigneten Medikamenten zur Vermeidung von Gefäßhyperproliferation beschichtet sein oder nach Bestrahlung oder Einbringung eines radioaktiven Materials in den Körper oder eine Beschichtung durch radioaktiven Zerfall Strahlung freisetzen.

Ferner kann die Gefäßstütze aus resorbierbaren Kunststoffen, z. B. aliphatischen Polyestern wie Polydioxanon, bestehen.

Soll die Gefäßstütze zur Schienung von Aneurysmen verwendet werden, wird sie vorzugsweise mit einem aufgenähten oder eingeflochtenen biokompatiblen Stoffgewebe aus Polyurethan, Silikon, Teflon oder Polyester versehen oder mit einer dünnwandigen Folie aus einem dieser Materialien vernäht, verschweißt, aufgeschrumpft oder verklebt.

Die rohrförmigen Körper aus Metall oder Kunststoff werden vorzugsweise aus nahtlos gezogenen Rohren gebildet, um Verspannungen und Risse zu vermeiden, wie das im Bereich von Schweißnähten der Fall wäre. Die Strukturen werden vorzugsweise durch Laserstrahl- oder Wasserstrahlschneiden, Elektroerosion und Elektropolitur hergestellt.

Nachfolgend werden Ausführungsbeispiele der Erfindung anhand einer Zeichnung weiter erläutert. In der Zeichnung zeigt:
- Fig. 1:: Eine bevorzugte Ausführungsform mit schlangenförmigen Ringelementen im Außenbereich, die durch hantelförmige Stege verbunden sind, und zickzackförmigen Ringelementen im Mittelbereich, die durch flexiblere s-förmige Stege verbunden sind,
- Fig. 2:: eine Variante der Ausführungsform von Figur 1, und
- Fig. 3:: verschiedene Formen von Biegeelementen.

Die in den Figuren dargestellten Gefäßstützen weisen alle neben schlangenlinien förmigen auch zickzackförmige Ringelemente mit S-förmig geformten Verbindungselementen auf. Aus Gründen der Übersichtlichkeit werden in den Figuren die abgerollten Gitterstrukturen der rohrförmigen Gefäßstützen dargestellt.

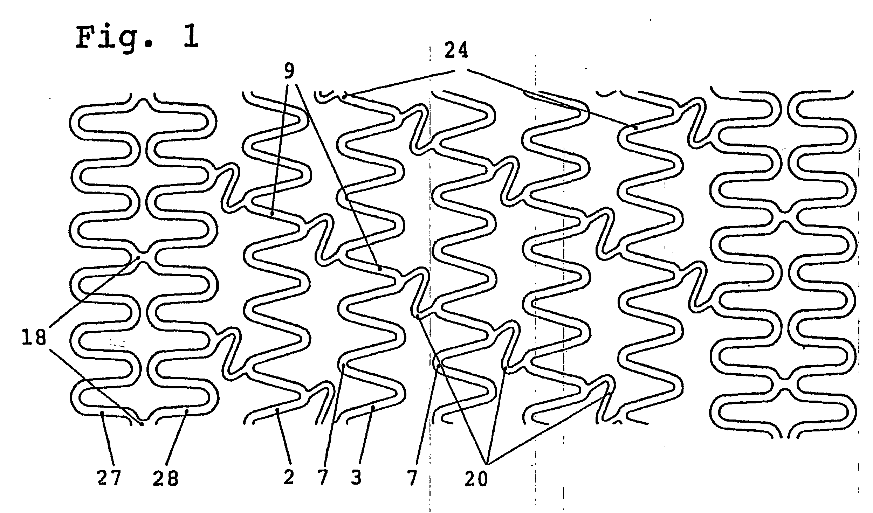

Figur 1 zeigt eine bevorzugte Ausführungsform der Erfindung mit jeweils zwei schlangenförmigen Ringelementen 27, 28 im Aussenbereich, die jeweils durch zwei hantelförmige Stege 18 verbunden sind, und zickzackförmigen Ringelementen 2, 3 im zentralen oder Mittelbereich, die durch jeweils zwei flexiblere S-förmige Biegeelemente 20 miteinander verbunden sind. Die S-förmigen Stege oder Biegeelemente 20 und die zickzackförmigen Ringelemente 2, 3 sind jeweils gleichsinnig angeordnet. Was die Ausrichtung der Bögen 7, 8 anbetrifft, verlaufen auch die Ringelemente 2 und 28 gleichsinnig, die Ringelemente 27 und 28 dagegen gegensinnig.

Dadurch wird eine große Flexibilität im Mittelbereich 24 erreicht. Als Besonderheit in dieser Darstellung sind die nach rechts geöffneten Bögen 7 der einzelnen Ringelemente 2, 3 jeweils auf gleicher Höhe angeordnet. Beim Aufdehnen dieser Struktur bilden die einzelnen sich dann aufdehnenden S-förmigen Stege 20 mit dazwischen liegenden einzelnen geraden Stegen 9 der zickzackförmigen Ringelemente 2, 3 jeweils eine umlaufende stabile Doppelhelixstruktur.

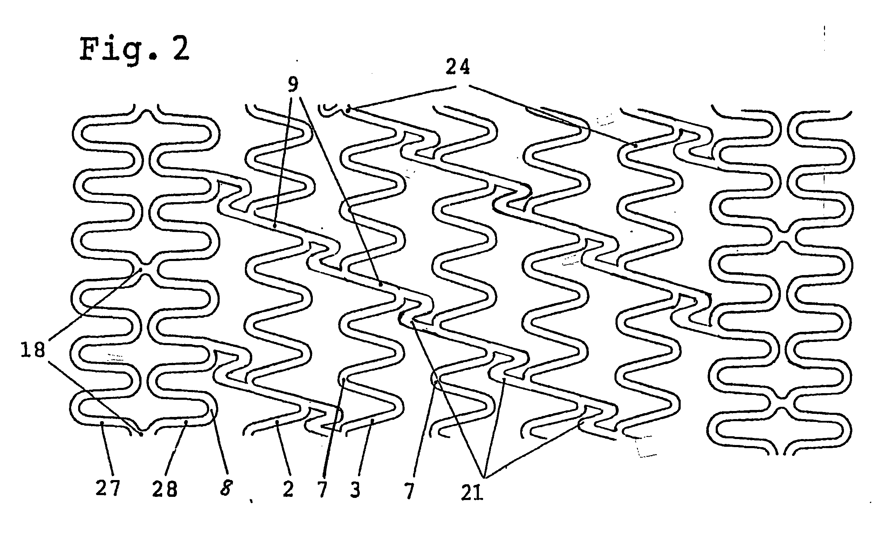

Figur 2 zeigt eine Variante des Design von Figur 1, bei der S-förmige Biegeelemente 21 gleichsinnig mit den Stegen 9 und Bögen 7 der zickzackförmigen Ringelemente 2, 3 verlaufen, d. h. sich die allgemeine Ausrichtung und der Verlauf der Ringelemente 2, 3 in den Biegeelementen 21 fortsetzt.

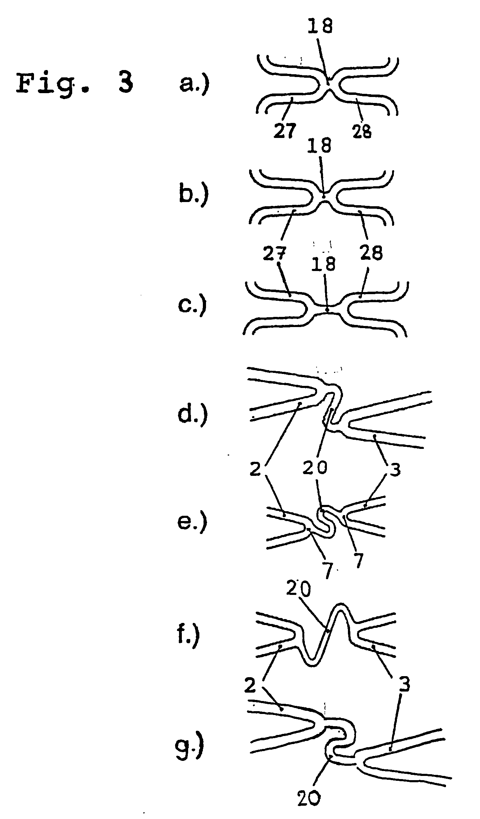

Figur 3 zeigt verschiedene Varianten von Biegeelementen 18, 21 und 22, wie sie zum Einsatz kommen können. Die hantelförmigen Biegeelemente 18 gemäß a), b) und c) unterscheiden sich durch ihr Länge zwischen den Bögen 8 der Ringelemente 27 und 28. Figur 3 d), e), f) und g) zeigen verschiedene Formen S-förmiger Biegeelemente 20 und 21. Es versteht sich, daß diese Formen alle auch spiegelbildlich ausgebildet sein können.

Aus der vorstehenden Beschreibung und der Darstellung von Ausführungsbeispielen wird deutlich, daß sich die Erfindung nicht auf die in den Ansprüchen oder der Beschreibung genannten Merkmalskombinationen beschränkt, sondern im Rahmen der Erfindung auch andere Kombinationen der aufgeführten Merkmale denkbar sind.

## Patentansprüche

1. Radial aufweitbare Gefäßstütze, welche eine Vielzahl von miteinander flexibel verbundenen Ringelementen (2, 3, 27, 28) aufweist, welche eine Gefäßstütze (1) mit einem proximalen und einem distalen Ende und einer Längsachse definieren, wobei die Ringelemente (2, 3, 27, 28) quer zur Längsachse der Gefäßstütze nebeneinander angeordnet sind, und jeweils durch Biegeelemente (18, 20, 21) miteinander verbunden sind, wobei
am proximalen und am distalen Ende der Gefäßstütze (1), jeweils wenigstens zwei randständige Ringelemente (27, 28) untereinander durch ein Paar hantelförmige Biegeelemente (18), d.h. um Wesentlichen gerade ausgerichteten Steg verbindungen verbunden sind und zentralständige Ringelemente (2, 3) untereinander-und mit randständigen Ringelementen (28) durch jeweils zwei S-förmige Biegeelemente (20, 21) verbunden sind, **dadurch gekennzeichnet, daß** zumindest die zentralständigen Ringelemente (2, 3) zickzackförmig ausgebildet sind.

2. Radial aufweitbare Gefäßstütze nach Anspruch 1, **dadurch gekennzeichnet, daß** die Biegeelemente (18, 20, 21) zwischen zwei Ringelementen (2, 3, 27, 28) jeweils ein Paar auf der Zirkumferenz gegenüberliegender Biegeelemente bilden.

3. Radial aufweitbare Gefäßstütze nach Anspruch 1 oder 2, **dadurch gekennzeichnet, daß** am proximalen und am distalen Ende der Gefäßstütze (1) jeweils zwei randständige Ringelemente (27, 28) vorhanden sind, die einen schlangenlinienförmigen Verlauf haben und an zueinander weisenden Bögen (8) die hantelförmigen Biegeelemente (18) aufweisen.

4. Radial aufweitbare Gefäßstütze nach einem der Ansprüche 1 bis 3, **dadurch gekennzeichnet, daß** die Breite der S-förmigen Stege (20, 21) 10 bis 50%, vorzugsweise etwa 30%, kleiner ist als die Breite von Stegen (9) der zickzackförmigen Ringelemente (2, 3).

5. Radial aufweitbare Gefäßstütze nach einem der Ansprüche 1 bis 4, **dadurch gekennzeichnet, daß** die zickzackförmigen Ringelemente (2, 3) an den Enden jeweils zu Bögen (7) abgerundet sind.

6. Radial aufweitbare Gefäßstütze nach einem der Ansprüche1 bis 5, **dadurch gekennzeichnet, daß** die Breite der zickzackförmigen Ringelement (2, 3) im Bereich von Bögen (7) größer ist als im Bereich von Stegen (9).

7. Radial aufweitbare Gefäßstütze nach einem der Ansprüche 1 bis 6, **dadurch gekennzeichnet, daß** die Breite von Stegen (9) und/oder Bögen (7) der zickzackförmigen Ringelemente (2, 3) an den seitlichen Enden der Gefäßstütze (1) größer ist als von denen im zentralen Bereich.

8. Radial aufweitbare Gefäßstütze nach einem der Ansprüche 1 bis 6, **dadurch gekennzeichnet, daß** die Breite von Stegen (9) und/oder der Bögen (7) der zickzackförmigen Ringelemente (2, 3) im zentralen Bereich der Gefäßstütze (1) größer ist als von denen an den Enden.

9. Radial aufweitbare Gefäßstütze nach einem der Ansprüche 1 bis 8, **dadurch gekennzeichnet, daß** die zickzackförmigen Ringelemente (2, 3) mit ihren Bögen (7) gleichsinnig ausgerichtet sind.

10. Radial aufweitbare Gefäßstütze nach einem der Ansprüche 1 bis 9, **dadurch gekennzeichnet, daß** die S-förmigen Biegeelemente (20, 21) diagonal über die Gefäßstütze (1) versetzt angeordnet sind, so daß sich eine Spiralform ausbildet.

11. Radial aufweitbare Gefäßstütze nach einem der Ansprüche 1 bis 10, **dadurch gekennzeichnet, daß** die S-förmigen Biegeelemente (20) zwischen den zickzackförmigen Ringelementen (2, 3) im wesentlichen in vertikaler Richtung angeordnet sind.

12. Radial aufweitbare Gefäßstütze nach einem der Ansprüche 1 bis 10, **dadurch gekennzeichnet, daß** die S-förmigen Biegeelemente (21) zwischen den zickzackförmigen Ringelementen (2, 3) im wesentlichen parallel zu Stegen (9) und Bögen (8) der zickzackförmigen Ringelemente (2, 3) ausgerichtet sind.

13. Radial aufweitbare Gefäßstütze nach einem der Ansprüche 1 bis 12, **dadurch gekennzeichnet, daß** sie im wesentlichen aus Stahl, Tantal, Titan, Niob, Platin oder einer Legierung aus mindestens einem dieser Metalle mit mindestens einem weiteren dieser oder anderer Metalle gebildet ist.

14. Radial aufweitbare Gefäßstütze nach Anspruch 13, **dadurch gekennzeichnet, daß** sie aus einer Nickel-Titan-Legierung besteht, die durch Wärmebehandlung selbstexpandierbar gemacht ist.

15. Radial aufweitbare Gefäßstütze nach einem der Ansprüche 1 bis 12, **dadurch gekennzeichnet, daß** sie aus einem resorbierbaren Stoff, vorzugsweise Kunststoff, besteht.

16. Radial aufweitbare Gefäßstütze nach einem der Ansprüche 1 bis 15, **dadurch gekennzeichnet, daß** sie mit einem biokompatiblen Material beschichtet ist.

17. Radial aufweitbare Gefäßstütze nach einem der Ansprüche 1 bis 16, **dadurch gekennzeichnet, daß** sie mit geeigneten Medikamenten zur Vermeidung einer Intimahyperproliferation der Gefäßwand beschichtet ist.

18. Radial aufweitbare Gefäßstütze nach Anspruch 17, **dadurch gekennzeichnet, daß** im Gebrauch die Beschichtung die Medikamente zur Vermeidung der Intimahyperproliferation der Gefäßwand langsam freisetzt.

19. Radial aufweitbare Gefäßstütze nach einem der Ansprüche 1 bis 16, **dadurch gekennzeichnet, daß** sie mit einem radioaktiven Material beschichtet ist, das eine radioaktive Strahlung freisetzt zur Vermeidung oder Reduktion von Intimahyperproliferation der Gefäßwand.

20. Radial aufweitbare Gefäßstütze nach Anspruch 16, **dadurch gekennzeichnet, daß** sie mit einem biokompatiblen Stoffgewebe aus Polyurethan, Silikon, Teflon oder Polyester oder einer dünnwandigen Folie aus einem dieser Materialien versehen ist.

## Claims

1. A radially expandable vessel support having a multitude of annular elements (2,3,27,28) flexibly connected to each other, defining a vascular support (1) with a proximal and a distal end and a longitudinal axis, wherein said annular elements (2,3,27,28) are arranged side by side transversely to said longitudinal axis of the vascular support and connected to each other by means of bending elements (18,20,21), wherein at the proximal and distal end of the vessel support (1) at least two marginally standing annular elements (27,28) each are linked among each other by a pair of dumbbell shaped bending elements (18), i.e. by bar connectors having a substantially straight configuration, and that centrally standing annular elements (2,3) are linked among each other and to marginally standing annular elements (28) by two S-shaped bending elements (20,21) each, **characterized in that** at least the centrally standing annular elements (2,3) are of a zigzag-shaped configuration.

2. A radially expandable vascular support pursuant to Claim 1, **characterized in that** the bending elements (18,20,21) between two annular elements (2,3,27,28) form a pair of bending elements lying opposite to each other on the circumference, respectively.

3. A radially expandable vascular support pursuant to one of the Claims 1 or 2, **characterized in that** at the proximal and distal end of said vascular support (1) there are two marginally standing annular elements (27,28) having a serpentine line-shaped course and having the dumbbell-shaped bending elements (18) at bows (8) pointing to each other.

4. A radially expandable vascular support pursuant to one of the Claims 1 to 3, **characterized in that** the width of the S-shaped bars (20,21) is by 10 to 50 %, preferably by about 30 % smaller than the width of bars (9) of the zigzag-shaped annular elements (2,3).

5. A radially expandable vascular support pursuant to one of the Claims 1 to 4, **characterized in that** the zigzag-shaped annular elements (2,3) are rounded-off at their ends to give them the shape of a bow (7).

6. A radially expandable vascular support pursuant to one of the Claims 1 to 5, **characterized in that** the width of the zigzag-shaped annular elements (2,3) in the area of bows (7) is larger than that in the area of bars (9).

7. A radially expandable vascular support pursuant to one of the Claims 1 to 6, **characterized in that** the width of bars (9) and/or bows (7) of the zigzag-shaped annular elements (2,3) at the lateral ends of the vascular support (1) is larger than that in the central area.

8. A radially expandable vascular support pursuant to one of the Claims 1 to 6, **characterized in that** the width of bars (9) and/or bows (7) of the zigzag-shaped annular elements (2,3) in the central area of the vascular support (1) is larger than that at the ends.

9. A radially expandable vascular support pursuant to one of the Claims 1 to 8, **characterized in that** the zigzag-shaped annular elements (2,3) with their bows (7) are equidirectionally orientated.

10. A radially expandable vascular support pursuant to one of the Claims 1 to 9, **characterized in that** the S-shaped bending elements (20,21) are diagonally staggered over the vascular support (1) so as to create a spiral shape.

11. A radially expandable vascular support pursuant to one of the Claims 1 to 10, **characterized in that** the S-shaped bending elements (20) between the zigzag-shaped annular elements (2,3) are mainly arranged in vertical direction.

12. A radially expandable vascular support pursuant to one of the Claims 1 to 10, **characterized in that** the S-shaped bending elements (21) between the zigzag-shaped annular elements (2,3) are mainly orientated in parallel to said bars (9) and bows (8) of the zigzag-shaped annular elements (2,3).

13. A radially expandable vascular support pursuant to one of the Claims 1 to 12, **characterized in that** it is essentially made of steel, tantalum, titanium, niobium, platinum, or of an alloy consisting of at least one of these metals and at least another one of these metals or other metals.

14. A radially expandable vascular support pursuant to Claim 13, **characterized in that** it is made of a nickel-titanium alloy which has been made self-expandable by way of thermal treatment.

15. A radially expandable vascular support pursuant to one of the Claims 1 to 12, **characterized in that** it is made of a resorbable material, preferably a plastic material.

16. A radially expandable vascular support pursuant to one of the Claims 1 to 15, **characterized in that** it is coated with a biocompatible material.

17. A radially expandable vascular support pursuant to one of the Claims 1 to 16, **characterized in that** it is coated with suitable medicaments to avoid intimate hyper proliferation of the vascular wall.

18. A radially expandable vascular support pursuant to Claim 17, **characterized in that** upon use the coating slowly releases the medicaments suitable to avoid intimate hyper proliferation of the vascular wall.

19. A radially expandable vascular support pursuant to one of the Claims 1 to 16, **characterized in that** it is coated with a radioactive material, which releases a radioactive radiation to avoid or reduce hyper proliferation of the vascular wall.

20. A radially expandable vascular support pursuant to one of the Claims 1 to 16, **characterized in that** it is provided with a biocompatible fabric made of polyurethane, silicone, Teflon, or polyester, or with a thin foil made of one of these materials.

## Revendications

1. Tuteur vasculaire radialement extensible, qui présente une pluralité d'éléments annulaires (2, 3, 27, 28) reliés de manière flexible les uns aux autres, qui définissent un tuteur vasculaire (1) avec une extrémité proximale et une extrémité distale et avec un axe longitudinal, les éléments annulaires (2, 3, 27, 28) étant placés les uns à côté des autres transversalement à l'axe longitudinal du tuteur vasculaire et étant reliés les uns aux autres à chaque fois par des éléments de flexion (18, 20, 21),
dans lequel, à l'extrémité proximale et à l'extrémité distale du tuteur vasculaire (1), à chaque fois au moins deux éléments annulaires de bordure (27, 28) sont reliés entre eux par une paire d'éléments de flexion (18) en forme d'haltère, c'est-à-dire de liaisons par barre orientées pour l'essentiel en ligne droite, et des éléments annulaires centraux (2, 3) sont reliés entre eux et avec des éléments annulaires de bordure (28) à chaque fois par deux éléments de flexion (20, 21) en forme de S, **caractérisé en ce qu'**au moins les éléments annulaires centraux (2, 3) sont réalisés en zigzag.

2. Tuteur vasculaire radialement extensible selon la revendication 1, **caractérisé en ce que** les éléments de flexion (18, 20, 21) entre deux éléments annulaires (2, 3, 27, 28) forment à chaque fois une paire d'éléments de flexion opposés sur la circonférence.

3. Tuteur vasculaire radialement extensible selon la revendication 1 ou 2, **caractérisé en ce qu'**à l'extrémité proximale et à l'extrémité distale du tuteur vasculaire (1) se trouvent à chaque fois deux éléments annulaires de bordure (27, 28) qui ont une forme sinueuse et présentent sur des courbes **(8)** en vis-à-vis les éléments de flexion **(18)** en forme d'haltères.

4. Tuteur vasculaire radialement extensible selon l'une des revendications 1 à 3, **caractérisé en ce que** la largeur des barres en S (20, 21) est jusqu'à 50 %, de préférence environ 30% inférieure à la largeur de barres (9) des éléments annulaires en zigzag (2, 3).

5. Tuteur vasculaire radialement extensible selon l'une des revendications 1 à 4, **caractérisé en ce que** les éléments annulaires en zigzag (2, 3) sont arrondis aux extrémités pour former des arcs (7).

6. Tuteur vasculaire radialement extensible selon l'une des revendications 1 à 5, **caractérisé en ce que** la largeur de l'élément annulaire en zigzag (2, 3) au niveau d'arcs (7) est plus grande qu'au niveau de barres (9).

7. Tuteur vasculaire radialement extensible selon l'une des revendications 1 à 6, **caractérisé en ce que** la largeur de barres (9) et/ou d'arcs (7) des éléments annulaires en zigzag (2, 3) au niveau des extrémités latérales du tuteur vasculaire (1) est supérieure à celle dans la partie centrale.

8. Tuteur vasculaire radialement extensible selon l'une des revendications 1 à 6, **caractérisé en ce que** la largeur des barres (9) et/ou des arcs (7) des éléments annulaires en zigzag (2, 3) est plus grande dans la partie centrale du tuteur vasculaire (1) qu'aux extrémités.

9. Tuteur vasculaire radialement extensible selon l'une des revendications 1 à 8, **caractérisé en ce que** les éléments annulaires en zigzag (2, 3) sont orientés dans le même sens avec leurs arcs (7).

10. Tuteur vasculaire radialement extensible selon l'une des revendications 1 à 9, **caractérisé en ce que** les éléments de flexion en S (20, 21) sont placés avec un décalage en diagonale sur le tuteur vasculaire (1), de sorte qu'on obtient une forme en spirale.

11. Tuteur vasculaire radialement extensible selon l'une des revendications 1 à 10, **caractérisé en ce que** les éléments de flexion en S (20) entre les éléments annulaires en zigzag (2, 3) sont placés pour l'essentiel dans la direction verticale.

12. Tuteur vasculaire radialement extensible selon l'une des revendications 1 à 10, **caractérisé en ce que** les éléments de flexion en S **(21)** entre les éléments annulaires en zigzag **(2, 3)** sont orientés pour l'essentiel parallèlement à des barres (9) et des arcs (8) des éléments annulaires en zigzag (2, 3).

13. Tuteur vasculaire radialement extensible selon l'une des revendications 1 à 12, **caractérisé en ce qu'**il est formé pour l'essentiel d'acier, de tantale, de titane, de niobium, de platine ou d'un alliage d'au moins l'un de ces métaux avec au moins un autre de ces métaux ou d'autres métaux.

14. Tuteur vasculaire radialement extensible selon la revendication 13, **caractérisé en ce qu'**il est composé d'un alliage de nickel et de titane, qui est rendu auto extensible par un traitement thermique.

15. Tuteur vasculaire radialement extensible selon l'une des revendications 1 à 12, **caractérisé en ce qu'**il est constitué d'un matériau résorbable, de préférence une matière plastique.

16. Tuteur vasculaire radialement extensible selon l'une des revendications 1 à 15, **caractérisé en ce qu'**il est revêtu d'un matériau biocompatible.

17. Tuteur vasculaire radialement extensible selon l'une des revendications 1 à 16, **caractérisé en ce qu'**il est revêtu de médicaments appropriés pour éviter une hyperprolifération intime de la paroi vasculaire.

18. Tuteur vasculaire radialement extensible selon la revendication 17, **caractérisé en ce qu'**en service, le revêtement libère lentement les médicaments pour éviter une hyperprolifération intime de la paroi vasculaire.

19. Tuteur vasculaire radialement extensible selon l'une des revendications 1 à 16, **caractérisé en ce qu'**il est revêtu d'un matériau radioactif qui libère un rayonnement radioactif afin d'éviter ou de réduire une hyperprolifération intime de la paroi vasculaire.

20. Tuteur vasculaire radialement extensible selon la revendication 16, **caractérisé en ce qu'**il est muni d'un tissu biocompatible en polyuréthanne, silicone, Téflon ou polyester ou d'une feuille mince de l'un de ces matériaux.
